# EUROPÄISCHE PATENTSCHRIFT

(11) **EP 4 370 044 B1**
(45) Veröffentlichungstag und Bekanntmachung des Hinweises auf die Patenterteilung: **09.10.2024**
(21) Anmeldenummer: 23754196.6
(22) Anmeldetag: 03.08.2023
(51) Int. Cl.: A61B 17/16

(54) **AUSRICHTEINHEIT UND MONTAGEVERFAHREN FÜR EIN MEDIZINISCHES INSTRUMENT**
ALIGNING UNIT AND ASSEMBLY METHOD FOR A MEDICAL INSTRUMENT
UNITÉ D'ALIGNEMENT ET PROCÉDÉ D'ASSEMBLAGE POUR UN INSTRUMENT MÉDICAL

(30) Priorität: 09.08.2022 DE 102022119981
(43) Veröffentlichungstag der Anmeldung: 22.05.2024
(73) Patentinhaber: Aesculap AG, 78532 Tuttlingen (DE)
(72) Erfinder: BUERK, Andre, 78056 Villingen-Schwenningen (DE); VOGLER, Aaron, 88637 Leibertingen (DE)
(74) Vertreter: Winter, Brandl - Partnerschaft mbB
(86) Internationale Anmeldenummer: PCT/EP2023/071588
(87) Internationale Veröffentlichungsnummer: WO 2024/033227

(56) Entgegenhaltungen:
- WO-A1-2006/111173
- DE-A1- 102015 110 415
- DE-A1- 19 945 322

## Beschreibung

### Technisches Gebiet

Die vorliegende Offenbarung betrifft eine Ausrichteinheit für ein medizinisches Instrument, insbesondere für ein Handinstrument. Ferner betrifft die vorliegende Offenbarung ein Montageverfahren für die Ausrichteinheit.

Bei medizinischen Instrumenten im Allgemeinen und Handinstrumenten im Speziellen ist es oftmals erforderlich, zwei schaftartige, beispielsweise zylinder-/hohlschaft-/hohlkörper-/hülsen-/rohrförmige, Bauteile miteinander zu verbinden, was auf einfache Weise über eine Gewindeverbindung erfolgen kann.

Ein Vorteil von solchen Gewindeverbindungen ist, dass die beiden zu verbindenden Bauteile stufenlos zueinander verstellt werden können, so dass sie zur radialen Ausrichtung in eine vorbestimmte Drehlage zueinander über eine Länge der Gewindeverbindung entweder weiter eingeschraubt/aufgeschraubt oder ausgeschraubt/abgeschraubt werden können. Eine radiale Ausrichtung ist insbesondere dann erforderlich, wenn eines der beiden Bauteile eine Nut, wie eine Einführnut, oder einen Vorsprung aufweist, die aufgrund äußerer Bauraumbedingungen an einer bestimmten Umfangsstelle angeordnet/ausgerichtet sein müssen.

Gleichzeitig liegt darin auch ein Nachteil von solchen Gewindeverbindungen, da diese, um mit einem vorbestimmten Drehmoment angezogen zu werden und somit gegen unbeabsichtigtes Lösen gesichert zu werden, axial an einem Widerlager anliegen müssen. Üblicherweise wird das axiale Anliegen dadurch erreicht, das eines der beiden zu verbindenden Bauteile vollständig in das andere der beiden zu verbindenden Bauteile eingeschraubt wird (oder auf das andere Bauteil aufgeschraubt wird), bis es an diesem anliegt. Jedoch ist bei einem vollständigen Einschrauben/Aufschrauben wiederum keine radiale Ausrichtung der beiden Bauteile möglich.

Stand der Technik diesbezüglich ist beispielsweise aus der DE 199 45 322 A1, WO 2006/111 173 A1, DE 10 2015 110 415 A1, DE 197 80 579 B4, US 2006/0 053 974 A1 oder US 6 050 989 A bekannt.

### Zusammenfassung der Offenbarung

Der vorliegenden Offenbarung liegt eine Aufgabe zugrunde, Nachteile des Stands der Technik zu reduzieren oder zu vermeiden. Insbesondere sollen eine Ausrichteinheit sowie ein Montageverfahren zum Montieren der Ausrichteinheit bereitgestellt werden, bei der zwei Bauteile gleichzeitig fest miteinander verbunden, insbesondere in einer beliebigen bzw. nicht fest vorgegebenen Axialposition axial fixiert, und radial zueinander ausgerichtet werden können. Zudem soll die Ausrichteinheit kompakt und kostengünstig aufgebaut sein sowie einfach zu montieren sein.

Die der vorliegenden Offenbarung zugrundeliegende Aufgabe wird durch eine Ausrichteinheit für ein medizinisches Instrument, insbesondere Handinstrument, mit den Merkmalen des Patentanspruchs 1 sowie durch ein Montageverfahren für die Ausrichteinheit mit den Merkmalen des nebengeordneten Patentanspruchs gelöst. Vorteilhafte Weiterbildungen sind Gegenstand der Unteransprüche und werden später genauer beschrieben.

Genauer ausgedrückt wird die Aufgabe durch eine Ausrichteinheit für ein medizinisches Instrument, insbesondere Handinstrument, gelöst, die ein Statorgehäuse, einen Gewindebolzen und einen Träger aufweist. In dem Statorgehäuse ist eine vorzugsweise als (Motor-)Handstück ausgebildete Antriebseinheit aufnehmbar. Vorzugsweise kann das Statorgehäuse einen (distalen) im Wesentlichen rohrförmigen Endabschnitt aufweisen. Der Gewindebolzen weist einen den Gewindebolzen mit dem Statorgehäuse (d.h. einem Innengewinde im Statorgehäuse) verbindenden Außengewindeabschnitt auf. Vorzugsweise kann der Gewindebolzen einen (proximalen) im Wesentlichen rohrförmigen Endabschnitt aufweisen, auf dessen Außenseite der Außengewindeabschnitt ausgebildet ist. Der Außengewindeabschnitt des Gewindebolzens kann in den Endabschnitt des Statorgehäuse eingeschraubt sein. Der Träger weist einen den Träger mit dem Statorgehäuse (d.h. einem Außengewinde im Statorgehäuse) verbindenden Innengewindeabschnitt auf. Vorzugsweise kann der Träger einen (proximalen) im Wesentlichen rohrförmigen Endabschnitt aufweisen, auf dessen Innenseite der Innengewindeabschnitt ausgebildet ist. Der Innengewindeabschnitt des Trägers kann auf den Endabschnitt des Statorgehäuse aufgeschraubt sein.

Mit anderen Worten weist die Ausrichteinheit das Statorgehäuse, den Gewindebolzen und den Träger auf, wobei sowohl der Gewindebolzen als auch der Träger über jeweils eine Gewindeverbindung an dem Statorgehäuse, insbesondere an dem distalen Endabschnitt des Statorgehäuse, befestigt sind/von proximaler Seite angeschraubt sind. Dabei kann der Gewindebolzen vorzugsweise innenseitig und der Träger außenseitig an dem Statorgehäuse angebracht sein. Alternativ könnte der Gewindebolzen auch außenseitig und der Träger innenseitig an dem Statorgehäuse angebracht sein. Das heißt, dass die Gewindeverbindungen des Gewindebolzens und des Trägers komplementär sind (d.h. innen ausgebildet und außen ausgebildet), aber nicht miteinander, sondern jeweils mit einer entsprechenden Gewindeverbindung am Statorgehäuse verschraubt werden können/sind.

Gemäß der vorliegenden Offenbarung sind der Außengewindeabschnitt des Gewindebolzens und der Innengewindeabschnitt des Trägers zueinander gegenläufig ausgebildet. Das heißt beispielsweise, dass der Gewindebolzen ein rechtslaufendes Gewinde und der Träger ein linkslaufendes Gewinde aufweist oder dass der Gewindebolzen ein linkslaufendes Gewinde und der Träger ein rechtslaufendes Gewinde aufweist. Dabei sind der Gewindebolzen und der Träger derart durch gegenläufige Drehbewegung/Kontern fixiert, dass der Träger und der Gewindebolzen axial aneinander anliegen, insbesondere gegeneinander verspannt sind. Dies hat den Vorteil, dass sich der Gewindebolzen zum Lösen in eine andere Drehrichtung als der Träger drehen müsste, so dass ein durch den axialen Kontakt zwischen dem Gewindebolzen und dem Träger entstehendes Reibmoment einem unbeabsichtigten Lösen der beiden Gewindeverbindungen entgegenwirkt. Gleichzeitig bleibt die stufenlose Einstellung über die Länge der Gewindeverbindungen und somit die Möglichkeit zur radialen Ausrichtung gegeben, da die axiale Position, an der der Gewindebolzen und der Träger aneinander anliegen nicht durch das Gewindeende fest vorgegeben ist, sondern über die Einschraubtiefe der beiden Gewindeverbindungen wie gewünscht/erforderlich festgelegt werden kann.

Die Aufgabe wird demnach insbesondere dadurch gelöst, dass sowohl der Gewindebolzen als auch der Träger über jeweils eine Gewindeverbindung/einen Gewindeabschnitt an dem Statorgehäuse angeschraubt sind, wobei der (Außen-) Gewindeabschnitt des Gewindebolzens und der (Innen-)Gewindeabschnitt des Trägers zueinander gegenläufig ausgebildet und derart durch gegenläufige Drehbewegung/Kontern fixiert/fixierbar sind, dass der Träger und der Gewindebolzen axial aneinander (an ihren Anschlagsflächen) anliegen, insbesondere gegeneinander verspannt sind. Dadurch können der Gewindebolzen und der Träger axial durch Verspannen fixiert werden, ohne dass eine (bspw. durch das Gewindeende bestimmte) Axialposition und damit auch eine Ausrichtung in einer Drehlage fest vorgegeben ist.

Vorzugsweise können das (Innen-)Gewinde des Statorgehäuses, an dem der Gewindebolzen anschraubbar ist, und das (Außen-)Gewinde des Statorgehäuses, an dem der Träger anschraubbar ist, zumindest teilweise in demselben Axialabschnitt des Statorgehäuses ausgebildet sein. Das heißt, dass der rohrförmige (End-)Abschnitt des Statorgehäuses außenseitig mit dem einen Bauteil, hier dem Träger, und innenseitig mit dem anderen Bauteil, hier dem Gewindebolzen, verschraubbar bzw. verschraubt ist. Mit anderen Worten sind die Gewindeverbindungen zwischen Statorgehäuse und Gewindebolzen und zwischen Statorgehäuse und Träger zumindest teilweise, vorzugsweise über die gesamte Axialerstreckung/Längserstreckung zumindest einer der (oder beider) Gewindeverbindungen, radial geschachtelt angeordnet. Anders ausgedrückt sind das Statorgehäuse, der Gewindebolzen und der Träger zumindest abschnittsweise radial geschachtelt angeordnet, wobei die Gewindeverbindungen zumindest teilweise, vorzugsweise zumindest eine der (oder beide) Gewindeverbindungen vollständig, in dem radial geschachtelten Bereich angeordnet sind.

Vorzugsweise weisen die Gewindeverbindung zwischen dem Statorgehäuse und dem Gewindebolzen und die Gewindeverbindung zwischen dem Statorgehäuse und dem Träger unterschiedliche Durchmesser/Nenngrößen auf. Dadurch wird eine radiale Schachtelung ermöglicht.

Gemäß einer bevorzugten Ausführungsform kann zwischen dem Statorgehäuse und dem Gewindebolzen ein erster axialer Spalt gebildet sein. Das heißt insbesondere, dass der Gewindebolzen nicht vollständig in das Statorgehäuse eingeschraubt ist bzw. ein weiteres Einschrauben durch ein axiales Anliegen des Gewindebolzens an dem Statorgehäuse unterbunden ist. Somit bleibt bezüglich des Gewindebolzens die Einstellbarkeit zur radialen Ausrichtung in beide Drehrichtungen gegeben.

Gemäß einer bevorzugten Ausführungsform kann zwischen dem Träger und dem Statorgehäuse ein zweiter axialer Spalt gebildet sein. Das heißt insbesondere, dass der Träger nicht vollständig auf das Statorgehäuse aufgeschraubt ist bzw. ein weiteres Aufschrauben durch ein axiales Anliegen des Trägers an dem Statorgehäuse unterbunden ist. Somit bleibt auch bezüglich des Trägers die Einstellbarkeit zur radialen Ausrichtung in beide Drehrichtungen gegeben.

Gemäß einer Weiterbildung der bevorzugten Ausführungsform kann die Ausrichteinheit ein Außengehäuse aufweisen. In dem Außengehäuse kann das Statorgehäuse aufgenommen sein. Ferner kann das Außengehäuse derart ausgebildet sein, dass es den zweiten axialen Spalt zwischen dem Träger und dem Statorgehäuse abdeckt. Somit wird vermieden, dass durch den außenseitig entstandenen zweiten Spalt Nachteile entstehen, wie beispielsweise, dass Verunreinigungen von außen in das Außengehäuse bzw. Statorgehäuse eindringen können.

Gemäß einer bevorzugten Ausführungsform kann der Gewindebolzen eine, vorzugsweise an seiner Innenseite ausgebildete, formschlüssige Werkzeugangriffsgeometrie, insbesondere in Form eines Innensechskants, zur Einleitung eines Drehmoments über einen ersten Steckschlüssel aufweisen. Dadurch kann der Gewindebolzen vorzugsweise von außen über den ersten Steckschlüssel in das Statorgehäuse eingeschraubt werden, ohne dass eine Demontage der Ausrichteinheit erforderlich ist.

Gemäß einer bevorzugten Ausführungsform kann der Träger eine, vorzugsweise an seiner Außenseite ausgebildete, formschlüssige Werkzeugangriffsgeometrie zur Einleitung eines Drehmoments über einen zweiten Steckschlüssel aufweisen. Dadurch kann der Träger vorzugsweise von außen über den zweiten Steckschlüssel auf das Statorgehäuse aufgeschraubt werden, ohne dass eine Demontage der Ausrichteinheit erforderlich ist.

Gemäß einer bevorzugten Ausführungsform kann die Ausrichteinheit eine separat von dem Statorgehäuse ausgebildete, fest, insbesondere drehfest und axialgesichert/axialfest, mit dem Statorgehäuse verbundene Gewindebuchse aufweisen. Die Gewindebuchse kann einen Innengewindeabschnitt ausbilden, über den der Außengewindeabschnitt des Gewindebolzens mit dem Statorgehäuse verbunden ist. Das heißt, dass das Statorgehäuse und der Gewindebolzen mittelbar über die als separates Einsetzteil ausgebildete, fest mit dem Statorgehäuse verbundene Gewindebuchse aneinander befestigt sind. Dies hat den Vorteil, dass die Gewindeverbindung zwischen dem Statorgehäuse und dem Gewindebolzen besonders kostengünstig hergestellt werden kann, da das Innengewinde an der vorgefertigten Gewindebuchse und nicht direkt, an dem unter Umständen schwer zur Bearbeitung zugänglichen oder schwer zu bearbeitenden Statorgehäuse direkt eingebracht werden muss.

Gemäß einer Weiterbildung der bevorzugten Ausführungsform kann die Gewindebuchse zumindest eine radial abstehende Nase, insbesondere radial nach außen abstehende, alternativ radial nach innen abstehende Nase, aufweisen. Beispielsweise kann die Gewindebuchse zwei über den Umfang gegenüberliegende Nasen aufweisen. Die Nase kann zur formschlüssig drehgesicherten und/oder formschlüssig axialgesicherten Verbindung mit dem Statorgehäuse in das Statorgehäuse, insbesondere eine entsprechende Aussparung in dem Statorgehäuse, eingreifen. Dies hat den Vorteil, dass auf besonders einfache Weise eine feste Verbindung zwischen der als Standardbauteil ausführbaren Gewindebuchse und dem Statorgehäuse realisiert werden kann. Zudem kann so eine geeignete Materialpaarung für die Gewindeverbindung gewählt werden, die unabhängig von der Materialwahl des Statorgehäuses ist.

Gemäß einer alternativen Ausführungsform kann das Statorgehäuse integral auf seiner Innenseite einen Innengewindeabschnitt ausbilden, über den der Außengewindeabschnitt des Gewindebolzens mit dem Statorgehäuse verbunden ist. Das heißt, dass der Innengewindeabschnitt auch direkt an dem Statorgehäuse/integral/werkstoffeinstückig mit dem Statorgehäuse ausgebildet sein kann. Dies kann insbesondere dann von Vorteil sein, wenn der Bauraum sehr begrenzt ist, so dass ein separates Einsetzteil nicht verwendet werden kann.

Gemäß einer bevorzugten Ausführungsform kann die Ausrichteinheit eine mit der Antriebseinheit drehmomentübertragend verbindbare Rotorwelle aufweisen. Beispielsweise kann über eine drehmomentübertragende Verbindung zu der drehantreibbaren Rotorwelle die Rotation der Rotorwelle direkt in eine Rotation eines Werkzeugs des Handinstruments oder indirekt in eine damit gekoppelte Bewegung, wie beispielsweise eine Abwinklung, des Werkzeugs umgewandelt werden. Die Rotorwelle kann abschnittsweise axial in den Gewindebolzen, insbesondere in einen axialen Bereich, in dem der Außengewindeabschnitt des Gewindebolzens oder die Werkzeugangriffsgeometrie des Gewindebolzens ausgebildet ist, hineinragen und auf ihrer Außenseite, insbesondere in dem axial hineinragenden Bereich, eine Werkzeugangriffsgeometrie, insbesondere in Form eines Außensechskants, zur Einleitung eines Drehmoments über einen Steckschlüssel, vorzugsweise über den ersten Steckschlüssel, aufweisen. Das heißt, dass die Werkzeugangriffsgeometrie der Rotorwelle und des Gewindebolzens vorzugsweise radial ineinander/radial geschachtelt angeordnet sind, so dass sie bei entsprechender Ausbildung des Steckschlüssels über denselben Steckschlüssel sowie ggf. gleichzeitig über den Steckschlüssel gedreht werden können. Dies hat den Vorteil, dass über den Steckschlüssel zugleich eine radiale Ausrichtung zwischen der Rotorwelle und dem Gewindebolzen bzw. eine drehfeste Fixierung beider Bauteile erreicht werden kann.

Gemäß einer Weiterbildung der bevorzugten Ausführungsform kann die Rotorwelle einen sich trichterartig verjüngenden Auslauf der Werkzeugangriffsgeometrie aufweisen. Das heißt, dass die Werkzeugangriffsgeometrie über radial angeschrägte Flächen in den Außendurchmesser der Rotorwelle übergeht. Dies hat den Vorteil, dass ein einfacheres Aufstecken des Steckschlüssels ermöglicht wird. Zudem wird ein automatisches Ausrichten der Rotorwelle zum Steckschlüssel sichergestellt, so dass der Steckschlüssel auch in dem durch die innenliegende Anordnung schwer einsehbaren Bereich der Rotorwelle verwendet werden kann.

Gemäß einer Weiterbildung der bevorzugten Ausführungsform kann die Rotorwelle, insbesondere an ihrem distalen Endabschnitt, einen Innengewindeabschnitt zur Aufnahme eines axialen Sicherungselements, das an dem Innengewindeabschnitt anschraubbar ist, aufweisen. Beispielsweise kann das axiale Sicherungselement als eine Gewindehülse ausgebildet sein, die auf ihrer Innenseite eine Einschraubgeometrie, insbesondere in Form eines Innensechskants, aufweist. Dadurch, dass in die Rotorwelle über den ersten Steckschlüssel ein Drehmoment eingeleitet werden kann, kann die Rotorwelle mittels des ersten Steckschlüssels festgehalten werden, wenn das axiale Sicherungselement in den Innengewindeabschnitt der Rotorwelle eingeschraubt wird. Somit kann die Rotorwelle von außen gegen Rotation gesichert werden und das axiale Sicherungselement, das wiederum vorzugsweise einen Sechskanteinsatz axial sichert, getauscht werden, ohne dass die Ausrichteinheit zerlegt werden muss. Somit können innerhalb der Rotorwelle angeordnete verschleißanfällige Teile, wie der Sechskanteinsatz einfach ausgewechselt werden.

Gemäß einer bevorzugten Ausführungsform kann die Ausrichteinheit einen ersten Steckschlüssel zur Einleitung des Drehmoments in die Werkzeugangriffsgeometrie des Gewindebolzens und zur Einleitung des Drehmoments in die Werkzeugangriffsgeometrie der Rotorwelle aufweisen. Der erste Steckschlüssel kann einen hohlzylindrischen/hohlkörperförmigen Angriffsabschnitt haben, auf dessen Außenseite einen zur der Werkzeugangriffsgeometrie des Gewindebolzens komplementären ersten Gegenabschnitt, insbesondere in Form eines Außensechskants, und auf dessen Innenseite einen zur der Werkzeugangriffsgeometrie der Rotorwelle komplementären zweiten Gegenabschnitt, insbesondere in Form eines Innensechskants, ausgebildet ist. Somit kann der erste Steckschlüssel sowohl zum Einleiten des Drehmoments in den Gewindebolzen sowie zum Einleiten des Drehmoments in die Rotorwelle bzw. zum Festhalten der Rotorwelle verwendet werden.

Gemäß einer Weiterbildung der bevorzugten Ausführungsform können der erste Gegenabschnitt und der zweite Gegenabschnitt vorzugsweise formgleich, d.h. insbesondere beide als ein Sechskant, insbesondere mit gleicher radialer Ausrichtung, ausgebildet sein. Dies hat den Vorteil, dass der Angriffsabschnitt mit einer verhältnismäßig geringen Wandstärke ausgebildet werden kann, so dass er platzsparend ist und auch in eine geringe radiale Lücke zwischen der Rotorwelle und dem Gewindebolzen eingeschoben werden kann.

Gemäß einer Weiterbildung der bevorzugten Ausführungsform kann der erste Steckschlüssel eine axiale Anschlagsfläche aufweisen, die so angeordnet ist, dass sowohl der erste Gegenabschnitt in Eingriff mit der Werkzeugangriffsgeometrie des Gewindebolzens ist als auch der zweite Gegenabschnitt in Eingriff mit der Werkzeugangriffsgeometrie der Rotorwelle ist, wenn die axiale Anschlagsfläche an dem Gewindebolzen oder an der Rotorwelle axial anliegt, d.h. wenn der erste Steckschlüssel im Steckeingriff ist/vollständig eingesteckt ist. Somit wird eine Begrenzung der Einschiebtiefe des ersten Steckschlüssels erreicht. Gleichzeitig kann bei vollständig eingestecktem ersten Steckschlüssel gewährleistet werden, dass über den ersten Steckschlüssel eine Drehkopplung zwischen der Rotorwelle und dem Gewindebolzen erreicht ist, so dass die Rotorwelle und der Gewindebolzen einfach fixiert/festgehalten werden können bzw. gleichzeitig das Drehmoment eingeleitet werden kann.

Die Aufgabe der vorliegenden Offenbarung wird auch durch ein medizinisches Instrument, insbesondere ein Handinstrument, gelöst, das die Ausrichteinheit aufweist.

Ferner wird die Aufgabe der vorliegenden Offenbarung durch ein Montageverfahren zum Montieren der Ausrichteinheit gelöst, wobei in einem ersten Schritt der Gewindebolzen in das Statorgehäuse eingeschraubt wird, bis der Gewindebolzen axial an dem Statorgehäuse anliegt, in einem zweiten Schritt der Träger auf das Statorgehäuse aufgeschraubt wird, bis der Träger axial an dem Gewindebolzen anliegt, in einem dritten Schritt der Träger so weit von dem Statorgehäuse abgeschraubt wird, bis der Träger relativ zu dem Statorgehäuse eine vorbestimmte radiale Ausrichtung hat, in einem vierten Schritt der Gewindebolzen so weit aus dem Statorgehäuse herausgeschraubt wird, bis der Gewindebolzen axial an dem Träger anliegt, und in einem fünften Schritt der Träger und der Gewindebolzen in gegenläufige Drehrichtungen zueinander verdreht werden.

Gemäß einer bevorzugten Ausführungsform des Montageverfahrens kann in einem dem ersten Schritt vorgelagerten Schritt die Gewindebuchse verdrehsicher und axialfest in das Statorgehäuse eingesetzt werden, wobei der Gewindebolzen in dem ersten Schritt zur Befestigung in dem Statorgehäuse in die Gewindebuchse eingeschraubt wird.

Gemäß einer bevorzugten Ausführungsform des Montageverfahrens kann in dem vierten Schritt, etwa über ein Hilfsmittel, ein vorbestimmter Winkelversatz, beispielsweise von 10 Grad, zu der vorbestimmten radialen Ausrichtung vorgehalten werden, so dass der vorbestimmte Winkelversatz durch das gegenläufige Verdrehen, d.h. durch ein vorbestimmtes Anzugsmoment beim Kontern, wieder ausgeglichen werden kann und nach dem fünften Schritt exakt die vorbestimmte radiale Ausrichtung vorliegt.

Mit anderen Worten betrifft die vorliegende Offenbarung eine Ausrichteinheit zur radialen Ausrichtung eines Trägers relativ zu einem Statorgehäuse. Dabei erfolgt eine stufenlose Verstellung durch ein Gewinde. Die Gewindeverbindung ist von außen einstellbar, so dass zur Verstellung keine Demontage erforderlich ist. Zudem wird ein linkslaufendes Gewinde verwendet, um die Verbindung gegen Lösen zu sichern. Insbesondere kann eine Gewindebuchse, etwa über zwei seitliche Nasen verdrehsicher im Statorgehäuse eingesetzt sein. Anschließend kann ein Gewindebolzen vollständig/komplett in die Gewindebuchse eingeschraubt werden. Ein Träger kann nun vollständig auf das Statorgehäuse aufgeschraubt werden, bis eine axiale Anschlagsfläche an dem Gewindebolzen erreicht ist. Zur radialen Ausrichtung des Trägers - insbesondere wenn eine am Umfang angeordnet Einführnut nicht in einer gewünschten/korrekten Position ist - kann der Träger danach so weit abgeschraubt werden, bis die gewünschte Ausrichtung des Trägers erreicht ist. Anschließend wird der Gewindebolzen herausgeschraubt/in Richtung distal geschraubt, bis die axiale Anschlagsfläche an dem Träger erreicht ist. Dadurch entsteht ein (axialer) Spalt zwischen dem Träger und dem Statorgehäuse, der jedoch durch ein Außengehäuse abgedeckt sein kann. Die endgültige Fixierung kann durch gegenläufige Drehbewegung von Gewindebolzen und Träger, d.h. durch Kontern, erfolgen.

Vorzugsweise kann ein Drehmoment am Gewindebolzen durch einen Innensechskantschlüssel eingeleitet werden und am Träger durch einen außenliegenden Steckschlüssel entgegengebracht werden. Dabei kann eine leichte Verdrehung beim Festschrauben durch entsprechendes Vorhalten eines Winkels kompensiert werden. Beispielsweise kann eine durch Hilfsmittel vorgegebene Verdrehung von 10 Grad realisiert sein, die sich beim Erreichen des vorgegebenen Drehmoments ausgleicht, so dass eine 0 Grad-Stellung erreicht wird.

Weiter kann die Ausrichteinheit zu einer Schnellwechselfunktion eines in der Rotorwelle aufgenommenen Sechskanteinsatzes verwendet werden. Dabei kann die Rotorwelle durch den doppelt vorhandenen Sechskant, d.h. den Innensechskant am Gewindebolzen und den Außensechskant auf der Rotorwelle, gegen Rotation gesichert werden und der Sechskanteinsatz getauscht werden, ohne dass dazu das Handstück/die Ausrichteinheit/das Instrument zerlegt werden müssen/muss. Dabei dient die Sechskantgeometrie des Sechskanteinsatzes zur Drehmomentübertragung von der Rotorwelle auf ein Werkzeug oder dergleichen des Instruments und ist vorzugsweise als ein separates Einsatzteil ausgebildet. Dieses separate, verschleißanfällige Einsatzteil kann einfach ausgewechselt werden, indem der Steckschlüssel in die Ausrichteinheit und auf die Rotorwelle gesteckt wird, um die Rotorwelle zu fixieren. Der Sechskanteinsatz ist üblicherweise axial durch eine Gewindehülse mit Innensechskant gesichert, die über ihren Innensechskant bei festgehaltener Rotorwelle gelöst werden kann.

### Kurzbeschreibung der Figuren

Fig. 1 ist eine Längsschnittdarstellung einer Ausrichteinheit gemäß der vorliegenden Offenbarung in einer vollständig montierten Position;
Fign. 2 bis 6 zeigen die Ausrichteinheit zu verschiedenen Zeitpunkten eines Montageverfahrens gemäß der Offenbarung; und
Fign. 7 bis 9 zeigen verschiedene Darstellungen der Ausrichteinheit und eines Steckschlüssels zum Einleiten von Drehmoment in einen Gewindebolzen und eine Rotorwelle der Ausrichteinheit.

### Beschreibung einer bevorzugten Ausführungsform

Nachstehend wird eine bevorzugte Ausführungsform der vorliegenden Offenbarung auf der Basis der zugehörigen Figuren beschrieben.

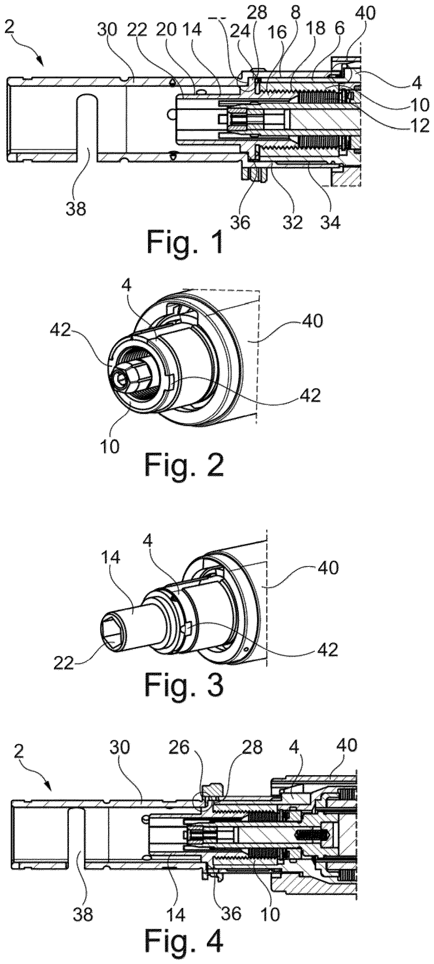

Fig. 1 zeigt eine Ausrichteinheit 2 für ein medizinisches Instrument, insbesondere ein Handinstrument, gemäß der vorliegenden Offenbarung in einer vollständig montierten Position.

Die Ausrichteinheit 2 weist ein Statorgehäuse 4 auf. Das Statorgehäuse 4 bildet insbesondere einen proximalen Bestandteil des medizinischen Instruments. In dem Statorgehäuse 4 ist eine vorzugsweise als (Motor-)Handstück ausgebildete (nicht dargestellte) Antriebseinheit aufnehmbar. Das Statorgehäuse 4 bildet einen Hohlraum zur Aufnahme der Antriebseinheit und anderer Bauteile des medizinischen Instruments aus. Das Statorgehäuse 4 weist an seinem distalen Ende einen im Wesentlichen rohrförmigen Endabschnitt/Hülsenabschnitt 6 auf. Der Endabschnitt 6 weist auf seiner Außenseite einen Außengewindeabschnitt 8 auf. Auf einer Innenseite des Endabschnitts 6 ist eine separat von dem Statorgehäuse 4 ausgebildete Gewindebuchse 10 aufgenommen, an der ein Innengewindeabschnitt 12 ausgebildet ist.

Die Ausrichteinheit 2 weist einen Gewindebolzen 14 auf. Der Gewindebolzen 14 ist distal des Statorgehäuses 4 angeordnet. Der Gewindebolzen 14 ist hohl ausgebildet/weist einen zentralen Durchgang auf. Der Gewindebolzen 14 weist an seinem proximalen Ende einen im Wesentlichen rohrförmigen Endabschnitt 16 auf. Der Endabschnitt 16 weist auf seiner Außenseite einen Außengewindeabschnitt 18 auf. Der Außengewindeabschnitt 18 des Gewindebolzens 14 ist komplementär zu dem Innengewindeabschnitt 12 der Gewindebuchse 10 ausgebildet. Der Gewindebolzen 14 ist über den Außengewindeabschnitt 18 mit dem Statorgehäuse 4 verschraubt/in das Statorgehäuse 4 eingeschraubt. Der Gewindebolzen 14 weist an seinem distalen Ende einen im Wesentlichen rohrförmigen Endabschnitt 20 auf. Der Endabschnitt 20 weist auf seiner Innenseite eine Werkzeugangriffsgeometrie 22 auf, über die ein Drehmoment (formschlüssig) in den Gewindebolzen 14 (von distaler Seite her) eingeleitet werden kann. Die Werkzeugangriffsgeometrie 22 ist in Form eines Innensechskants ausgebildet. Der Gewindebolzen 14 weist einen radial nach außen abstehenden Vorsprung 24 auf. In der dargestellten Ausführungsform ist der Vorsprung 24 in Form eines umlaufenden Flansches ausgebildet. An dem Vorsprung 24 ist distal (auf einer dem Statorgehäuse 4 abgewandten Axialseite) eine erste axiale Anschlagsfläche 26 ausgebildet. An dem Vorsprung 24 ist proximal (auf einer dem Statorgehäuse 4 zugewandten Axialseite) eine zweite axiale Anschlagsfläche 28 ausgebildet. In dem vollständig montierten Zustand ist ein erster axialer Spalt zwischen der zweiten axialen Anschlagsfläche 28 und dem Statorgehäuse 4 bzw. der Gewindebuchse 10 ausgebildet, d.h., dass der Gewindebolzen 14 nicht vollständig/bis zum Anschlag in die Gewindebuchse 10 eingeschraubt ist.

Die Ausrichteinheit 2 weist einen Träger 30 auf. Der Träger 30 ist distal des Statorgehäuses 4 angeordnet. Der Träger 30 ist hohl ausgebildet/weist einen zentralen Durchgang auf. Der Träger 30 weist an seinem proximalen Ende einen im Wesentlichen rohrförmigen Endabschnitt 32 auf. Der Endabschnitt 32 weist auf seiner Innenseite einen Innengewindeabschnitt 34 auf. Der Innengewindeabschnitt 34 des Trägers 30 ist komplementär zu dem Außengewindeabschnitt 8 des Statorgehäuses 4 ausgebildet. Der Träger 30 ist über den Innengewindeabschnitt 34 mit dem Statorgehäuse 4 verschraubt/auf das Statorgehäuse 4 aufgeschraubt. Der Träger 30 weist auf seiner Außenseite eine (nicht explizit dargestellte) Werkzeugangriffsgeometrie auf, über die ein Drehmoment (formschlüssig) in den Träger 30 (von außen her) eingeleitet werden kann. Distal des Innengewindeabschnitts 34 verjüngt sich der Träger 30 über eine radiale Stufe, durch die eine axiale Anlagefläche 36 gebildet wird. Die axiale Anlagefläche 36 liegt im montierten Zustand an der ersten axialen Anschlagsfläche 26 des Gewindebolzens 14 an. In dem vollständig montierten Zustand ist ein zweiter axialer Spalt zwischen dem Endabschnitt 32 und dem Statorgehäuse 4 ausgebildet, d.h., dass der Träger 30 nicht vollständig/bis zum Anschlag auf das Statorgehäuse 4 aufgeschraubt ist. Der Träger 30 weist eine Einführnut 38 auf, die auf dem Umfang des Trägers 30 (hier als Durchgangsloch) ausgebildet ist und sich in Axialrichtung des Trägers 30 erstreckt. Die Einführnut 38 ist in dem montierten Zustand der Ausrichteinheit 2 in einer vorbestimmten Ausrichtung/Umfangsposition/Drehlage angeordnet (in der dargestellten Ausführungsform, so dass sie unten/in einer 0°-Stellung angeordnet ist).

Vorzugsweise kann die Ausrichteinheit 2 ein Außengehäuse 40 aufweisen, in dem das Statorgehäuse 4 angeordnet ist, das derart ausgebildet ist, dass es den zweiten axialen Spalt zwischen dem Träger 30 und dem Statorgehäuse 4 abdeckt.

Gemäß der vorliegenden Offenbarung sind der Außengewindeabschnitt 18 des Gewindebolzens 14 und der Innengewindeabschnitt 34 des Trägers 30 gegenläufig ausgebildet. Das heißt, dass der Gewindebolzen 14 und der Träger 30 in unterschiedliche Drehrichtungen gedreht werden müssen, um weiter aufgeschraubt/eingeschraubt bzw. abgeschraubt/herausgeschraubt zu werden. Zudem sind der Gewindebolzen 14 und der Träger 30 durch gegenläufige Drehbewegung derart fixiert/gekontert, dass der Träger 30 und der Gewindebolzen 14 axial verspannt aneinander anliegen.

Ein Montageverfahren zum Montieren der Ausrichteinheit 2 wird nachfolgend erläutert:
In einem dem Montageverfahren vorgelagerten Schritt, der in Fig. 2 dargestellt ist, wird die Gewindebuchse 10 in das Statorgehäuse 4 eingesetzt. Die Gewindebuchse 10 weist zwei radial nach außen abstehende Nasen 42 auf, die in entsprechende Aussparungen in dem Statorgehäuse 4 eingreifen, so dass die Gewindebuchse 10 verdrehsicher und axialfest mit dem Statorgehäuse 4 verbunden ist.

In einem ersten Schritt des Montageverfahrens, der in Fig. 3 dargestellt ist, wird der Gewindebolzen 14 in das Statorgehäuse 4 (bzw. in die Gewindebuchse 10) eingeschraubt. Der Gewindebolzen 14 wird vollständig in das Statorgehäuse 4 eingeschraubt d.h. so weit, bis der Gewindebolzen 14 axial an dem Statorgehäuse 4 (bzw. der Gewindebuchse 10) anliegt. Die Drehmomenteinleitung in den Gewindebolzen 14 erfolgt dabei insbesondere über die Werkzeugangriffsgeometrie 22 in Form des Innensechskants.

In einem zweiten Schritt des Montageverfahren, der in Fig. 4 dargestellt ist, wird der Träger 30 auf das Statorgehäuse 4 aufgeschraubt. Der Träger 30 wird vollständig auf das Statorgehäuse 4 aufgeschraubt d.h. so weit, bis die axiale Anlagefläche 36 an der ersten axialen Anschlagsfläche 26 anliegt. Die Drehmomenteinleitung in den Träger 30 erfolgt dabei insbesondere über die außenliegende (nicht explizit dargestellte) Werkzeugangriffsgeometrie des Trägers 30.

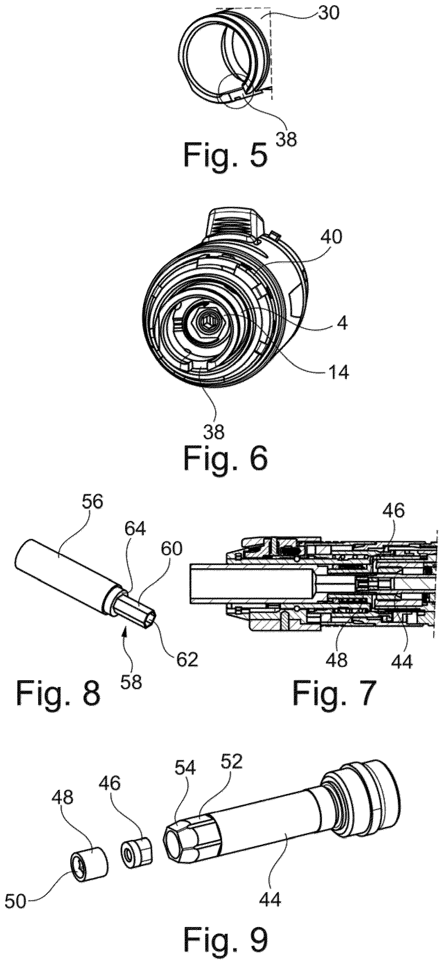

In der Regel befindet sich die Einführnut 38 des Trägers 30, wenn der Träger 30 vollständig aufgeschraubt wurde, nicht in der gewünschten radialen Ausrichtung (vgl. Fig. 5). Sollte dies der Fall sein, wird der Träger 30 in einem dritten Schritt des Montageverfahrens so weit von dem Statorgehäuse 4 abgeschraubt, bis der Träger 30 (insbesondere die Einführnut 38) relativ zu dem Statorgehäuse 4 die vorbestimmte radiale Ausrichtung hat (vgl. Fig. 1). Anschließend wird der Gewindebolzen 14 in einem vierten Schritt des Montageverfahrens so weit aus dem Statorgehäuse 4 herausgeschraubt, bis der Gewindebolzen 14 axial mit seiner ersten Anschlagsfläche 26 an der axialen Anlagefläche des Trägers 30 anliegt (vgl. Fig. 6 und Fig. 1)

In einem fünften Schritt des Montageverfahrens werden der Träger 30 und der Gewindebolzen 14 in gegenläufige Drehrichtungen zueinander verdreht/gekontert, so dass sie mit einem vorbestimmten Drehmoment angezogen/gegeneinander verspannt werden.

Dabei kann eine leichte Verdrehung beim Festschrauben/Kontern durch entsprechendes Vorhalten eines Winkels, etwa durch ein Hilfsmittel, kompensiert werden, wobei der Winkel bei Erreichen des vorbestimmten Drehmoments ausgeglichen wird.

Gemäß einem weiteren Aspekt der vorliegenden Offenbarung weist die Ausrichteinheit 2 eine mit der Antriebseinheit drehmomentübertragend verbindbare Rotorwelle 44 auf (vgl. Fign. 7 bis 9). Die Rotorwelle 44 ragt abschnittsweise axial in den Gewindebolzen 14, insbesondere in einen axialen Bereich, in dem der Außengewindeabschnitt 18 des Gewindebolzens 14 oder die Werkzeugangriffsgeometrie 22 des Gewindebolzens 14 ausgebildet ist, hinein.

Beispielsweise kann über eine drehmomentübertragende Verbindung zu der drehantreibbaren Rotorwelle 44 die Rotation der Rotorwelle 44 direkt in eine Rotation eines (nicht dargestellten) Werkzeugs des Instruments/Handinstruments oder indirekt in eine damit gekoppelte Bewegung, wie beispielsweise eine Abwinklung, des Werkzeugs umgewandelt werden.

Dazu ist in der Rotorwelle 44 ein Sechskanteinsatz 46 drehfest aufgenommen, über den die Rotation der Rotorwelle 44 weiterübertragen werden kann. Der Sechskanteinsatz 46 ist als ein Einsatzteil ausgebildet und weist auf seiner Innenseite einen Innensechskant zur Drehmomentweitergabe auf. Zur axialen Sicherung des Sechskanteinsatzes 46 ist in der Rotorwelle 44 zudem ein Sicherungselement in Form einer Gewindehülse 48 aufgenommen, welche axial an dem Sechskanteinsatz 46 anliegt und somit ein Herausfallen des Sechskanteinsatzes 46 aus der Rotorwelle verhindert.

Die Gewindehülse 48 weist auf ihrer Außenseite ein Außengewinde auf, über das sie mit einem entsprechend ausgebildeten Innengewinde an der Innenseite der Rotorwelle 44 verschraubt ist. Zur Einleitung eines Drehmoments in die Gewindehülse 48 weist die Gewindehülse 48 einen Innensechskant 50 auf.

Die Rotorwelle 44 weist auf ihrer Außenseite, insbesondere in dem axial in den Gewindebolzen 14 hineinragenden Bereich, eine Werkzeugangriffsgeometrie 52, insbesondere in Form eines Außensechskants, zur Einleitung eines Drehmoments auf. Vorzugsweise weist die Rotorwelle 44 einen sich trichterartig verjüngenden Auslauf 54 der Werkzeugangriffsgeometrie 52, etwa in Form radial angeschrägter Flächen des Außensechskants, auf.

Ferner weist die Ausrichteinheit 2 einen Steckschlüssel 56 auf. Der Steckschlüssel 56 dient zur Einleitung des Drehmoments in die Werkzeugangriffsgeometrie 22 des Gewindebolzens 14 und zur Einleitung des Drehmoments in die Werkzeugangriffsgeometrie 52 der Rotorwelle 44. Der Steckschlüssel 56 weist einen hohlzylindrischen/hohlkörperförmigen Angriffsabschnitt 58 auf, auf dessen Außenseite ein zur der Werkzeugangriffsgeometrie 22 des Gewindebolzens 14 komplementärer erster Gegenabschnitt 60, insbesondere in Form eines Außensechskants, und auf dessen Innenseite ein zur der Werkzeugangriffsgeometrie 52 der Rotorwelle 44 komplementärer zweiter Gegenabschnitt 62, insbesondere in Form eines Innensechskants, ausgebildet ist.

Vorzugsweise können der erste Gegenabschnitt 60 und der zweite Gegenabschnitt 62 formgleich, d.h. insbesondere beide als ein Sechskant, insbesondere mit gleicher radialer Ausrichtung, ausgebildet sein. Weiter kann der Steckschlüssel 56 eine axiale Anschlagsfläche 64 aufweisen, die so angeordnet ist, dass sowohl der erste Gegenabschnitt 60 in Eingriff mit dem Innensechskant des Gewindebolzens 14 ist als auch der zweite Gegenabschnitt 62 in Eingriff mit dem Außensechskant 52 der Rotorwelle 44 ist, wenn die axiale Anschlagsfläche 64 an dem Gewindebolzen 14 axial anliegt, d.h. vollständig eingesteckt ist (vgl. Fig. 7).

### Bezugszeichenliste

- 2: Ausrichteinheit
- 4: Statorgehäuse
- 6: rohrförmiger Endabschnitt
- 8: Außengewindeabschnitt
- 10: Gewindebuchse
- 12: Innengewindeabschnitt
- 14: Gewindebolzen
- 16: rohrförmiger Endabschnitt
- 18: Außengewindeabschnitt
- 20: rohrförmiger Endabschnitt
- 22: Werkzeugangriffsgeometrie
- 24: Vorsprung
- 26: erste axiale Anschlagsfläche
- 28: zweite axiale Anschlagsfläche
- 30: Träger
- 32: rohrförmiger Endabschnitt
- 34: Innengewindeabschnitt
- 36: axiale Anlagefläche
- 38: Einführnut
- 40: Außengehäuse
- 42: radial abstehende Nase
- 44: Rotorwelle
- 46: Sechskanteinsatz
- 48: Gewindehülse
- 50: Innensechskant
- 52: Werkzeugangriffsgeometrie
- 54: trichterartig verjüngender Auslauf
- 56: erster Steckschlüssel
- 58: hohlkörperförmiger Angriffsabschnitt
- 60: erster Gegenabschnitt
- 62: zweiter Gegenabschnitt
- 64: axiale Anschlagsfläche

## Patentansprüche

1. Ausrichteinheit (2) für ein medizinisches Instrument, insbesondere Handinstrument,
zur radialen Ausrichtung zweier bis zu einer gegenseitigen Axialanlage anzuschraubender Bauteile (14, 30),
mit einem rohrförmigen Statorgehäuse (4), in dem eine vorzugsweise als Handstück ausgebildete Antriebseinheit aufnehmbar ist, einem Gewindebolzen (14), der einen den Gewindebolzen (14) mit dem Statorgehäuse (4) verbindenden Außengewindeabschnitt (18) aufweist, und einem rohrförmigen Träger (30), der einen den Träger (30) mit dem Statorgehäuse (4) verbindenden Innengewindeabschnitt (34) aufweist, wobei das Statorgehäuse (4), der Gewindebolzen (14) und der Träger (30) eine gemeinsame Längsachse haben,
**dadurch gekennzeichnet, dass**
der Außengewindeabschnitt (18) des Gewindebolzens (14) und der Innengewindeabschnitt (34) des Trägers (30) gegenläufig ausgebildet sind und durch gegenläufige Drehbewegung derart fixiert sind, dass der Träger (30) und der Gewindebolzen (14) axial aneinander anliegen.

2. Ausrichteinheit (2) nach Anspruch 1, **dadurch gekennzeichnet, dass** zwischen dem Statorgehäuse (4) und dem Gewindebolzen (14) ein erster axialer Spalt gebildet ist.

3. Ausrichteinheit (2) nach Anspruch 1 oder 2, **dadurch gekennzeichnet, dass** zwischen dem Träger (30) und dem Statorgehäuse (4) ein zweiter axialer Spalt gebildet ist und die Ausrichteinheit (2) ein Außengehäuse (40) aufweist, in dem das Statorgehäuse (4) aufgenommen ist, und das Außengehäuse (40) derart ausgebildet ist, dass es den zweiten axialen Spalt zwischen dem Träger (30) und dem Statorgehäuse (4) abdeckt.

4. Ausrichteinheit (2) nach einem der Ansprüche 1 bis 3, **dadurch gekennzeichnet, dass** der Gewindebolzen (14) eine, vorzugsweise an seiner Innenseite ausgebildete, Werkzeugangriffsgeometrie (22), insbesondere in Form eines Innensechskants, zur Einleitung eines Drehmoments über einen ersten Steckschlüssel (56) aufweist und/oder der Träger (30) eine, vorzugsweise an seiner Außenseite ausgebildete, Werkzeugangriffsgeometrie zur Einleitung eines Drehmoments über einen zweiten Steckschlüssel aufweist.

5. Ausrichteinheit (2) nach einem der Ansprüche 1 bis 4, **dadurch gekennzeichnet, dass** die Ausrichteinheit (2) eine separat von dem Statorgehäuse (4) ausgebildete, fest mit dem Statorgehäuse (4) verbundene Gewindebuchse (10) aufweist, die einen Innengewindeabschnitt (12) ausbildet, über den der Außengewindeabschnitt (18) des Gewindebolzens (14) mit dem Statorgehäuse (4) verbunden ist.

6. Ausrichteinheit (2) nach Anspruch 5, **dadurch gekennzeichnet, dass** die Gewindebuchse (10) zumindest eine radial abstehende Nase (42) aufweist, die zur formschlüssig drehgesicherten und/oder formschlüssig axialgesicherten Verbindung mit dem Statorgehäuse (4) in das Statorgehäuse (4), insbesondere eine entsprechende Aussparung in dem Statorgehäuse (4), eingreift.

7. Ausrichteinheit (2) nach einem der Ansprüche 1 bis 6, **dadurch gekennzeichnet, dass** die Ausrichteinheit (2) eine mit der Antriebseinheit drehmomentübertragend verbindbare Rotorwelle (44) aufweist, die abschnittsweise axial in den Gewindebolzen (14) hineinragt und auf ihrer Außenseite eine Werkzeugangriffsgeometrie (52), insbesondere in Form eines Außensechskants, zur Einleitung eines Drehmoments über einen Steckschlüssel, vorzugsweise über den ersten Steckschlüssel (56), aufweist.

8. Ausrichteinheit (2) nach Anspruch 7, **dadurch gekennzeichnet, dass** die Rotorwelle (44) einen sich trichterartig verjüngenden Auslauf der Werkzeugangriffsgeometrie (54) aufweist.

9. Ausrichteinheit (2) nach Anspruch 7 oder 8, **dadurch gekennzeichnet, dass** die Ausrichteinheit (2) einen ersten Steckschlüssel (56) zur Einleitung des Drehmoments in die Werkzeugangriffsgeometrie (22) des Gewindebolzens (14) und zur Einleitung des Drehmoments in die Werkzeugangriffsgeometrie (52) der Rotorwelle (44) aufweist, der einen hohlkörperförmigen Angriffsabschnitt (58) hat, auf dessen Außenseite einen zu der Werkzeugangriffsgeometrie (22) des Gewindebolzens (14) komplementären ersten Gegenabschnitt (60), insbesondere in Form eines Außensechskants, und auf dessen Innenseite einen zu der Werkzeugangriffsgeometrie (52) der Rotorwelle (44) komplementären zweiten Gegenabschnitt (62), insbesondere in Form eines Innensechskants, ausgebildet ist.

10. Montageverfahren zum Montieren einer Ausrichteinheit (2) für ein medizinisches Instrument nach einem der Ansprüche 1 bis 9, wobei
- der Gewindebolzen (14) in das Statorgehäuse (4) eingeschraubt wird, bis der Gewindebolzen (14) axial an dem Statorgehäuse (4) anliegt,
- der Träger (30) auf das Statorgehäuse (4) aufgeschraubt wird, bis der Träger (30) axial an dem Gewindebolzen (14) anliegt,
- der Träger (30) so weit von dem Statorgehäuse (4) abgeschraubt wird, bis der Träger (30) relativ zu dem Statorgehäuse (4) eine vorbestimmte radiale Ausrichtung hat,
- der Gewindebolzen (14) so weit aus dem Statorgehäuse (4) herausgeschraubt wird, bis der Gewindebolzen (14) axial an dem Träger (30) anliegt, und
- der Träger (30) und der Gewindebolzen (14) in gegenläufige Drehrichtungen zueinander verdreht werden.

## Claims

1. An alignment unit (2) for a medical instrument, in particular a hand instrument,
for the radial alignment of two components (14, 30) to be screwed on up to a mutual axial contact,
including a tubular stator housing (4), in which a drive unit preferably in the form of a handpiece can be accommodated, a threaded bolt (14) having an external threaded portion (18) that connects the threaded bolt (14) to the stator housing (4), and a tubular carrier (30) having an internal threaded portion (34) that connects the carrier (30) to the stator housing (4), the stator housing (4), the threaded bolt (14) and the carrier (30) having a common longitudinal axis,
**characterized in that**
the external threaded portion (18) of the threaded bolt (14) and the internal threaded portion (34) of the carrier (30) are formed in opposite directions and are fixed by counter-rotating movement in such a way that the carrier (30) and the threaded bolt (14) lie axially against each other.

2. The alignment unit (2) according to claim 1, **characterized in that** a first axial gap is formed between the stator housing (4) and the threaded bolt (14).

3. The alignment unit (2) according to claim 1 or 2, **characterized in that** a second axial gap is formed between the carrier (30) and the stator housing (4), and the alignment unit (2) has an outer housing (40), in which the stator housing (4) is accommodated, and the outer housing (40) is configured in such a way that it covers the second axial gap between the carrier (30) and the stator housing (4).

4. The alignment unit (2) according to any of claims 1 to 3, **characterized in that** the threaded bolt (14) has a tool engagement geometry (22), preferably formed on its inner side, in particular in the form of an internal hexagon, for introducing a torque via a first socket wrench (56), and/or the carrier (30) has a tool engagement geometry, preferably formed on its outer side, for introducing a torque via a second socket wrench.

5. The alignment unit (2) according to any of claims 1 to 4, **characterized in that** the alignment unit (2) has a threaded bush (10) which is formed separately from the stator housing (4), is firmly connected to the stator housing (4) and forms an internal threaded portion (12) via which the external threaded portion (18) of the threaded bolt (14) is connected to the stator housing (4).

6. The alignment unit (2) according to claim 5, **characterized in that** the threaded bush (10) has at least one radially projecting lug (42) which engages in the stator housing (4), in particular in a corresponding recess in the stator housing (4), for a positive-locking rotationally secured and/or positive-locking axially secured connection to the stator housing (4).

7. The alignment unit (2) according to any of claims 1 to 6, **characterized in that** the alignment unit (2) has a rotor shaft (44) that can be connected to the drive unit in a torque-transmitting manner, projects axially in sections into the threaded bolt (14) and has on its outside a tool engagement geometry (52), in particular in the form of an external hexagon for introducing a torque via a socket wrench, preferably via the first socket wrench (56).

8. The alignment unit (2) according to claim 7, **characterized in that** the rotor shaft (44) has a funnel-like tapering outlet of the tool engagement geometry (54).

9. The alignment unit (2) according to claim 7 or 8, **characterized in that** the alignment unit (2) has a first socket wrench (56) for introducing the torque into the tool engagement geometry (22) of the threaded bolt (14), and for introducing the torque into the tool engagement geometry (52) of the rotor shaft (44), which has a hollow body-shaped engagement portion (58), on the outside of which is formed a first mating portion (60) complementary to the tool engagement geometry (22) of the threaded bolt (14), in particular in the form of an external hexagon, and on the inside of which is formed a second mating portion (62) complementary to the tool engagement geometry (52) of the rotor shaft (44), in particular in the form of an internal hexagon.

10. A mounting method for mounting an alignment unit (2) for a medical instrument according to any of claims 1 to 9, wherein
- the threaded bolt (14) is screwed into the stator housing (4) until the threaded bolt (14) is in axial contact with the stator housing (4),
- the carrier (30) is screwed onto the stator housing (4) until the carrier (30) is in axial contact with the threaded bolt (14),
- the carrier (30) is unscrewed from the stator housing (4) until the carrier (30) has a predetermined radial alignment relative to the stator housing (4),
- the threaded bolt (14) is unscrewed from the stator housing (4) until the threaded bolt (14) is in axial contact with the carrier (30), and
- the carrier (30) and the threaded bolt (14) are turned in opposite directions of rotation to each other.

## Revendications

1. Unité d'alignement (2) pour un instrument médical, en particulier un instrument à main,
pour l'alignement radial de deux composants (14, 30) à visser jusqu'à un appui axial mutuel,
avec un boîtier de stator (4) tubulaire dans lequel une unité d'entraînement conçue de préférence en tant que pièce à main peut être reçue, un boulon fileté (14) qui présente une section de filetage extérieur (18) connectant le boulon fileté (14) au boîtier de stator (4) et un support (30) tubulaire qui présente une section de filetage intérieur (34) connectant le support (30) au boîtier de stator (4), dans laquelle le boîtier de stator (4), le boulon fileté (14) et le support (30) présentent un axe longitudinal commun,
**caractérisée en ce que**
la section de filetage extérieur (18) du boulon fileté (14) et la section de filetage intérieur (34) du support (30) sont conçues en sens inverse et sont fixées par un mouvement de rotation en sens inverse, de sorte que le support (30) et le boulon fileté (14) s'appliquent axialement l'un contre l'autre.

2. Unité d'alignement (2) selon la revendication 1, **caractérisée en ce qu'**une première fente axiale est formée entre le boîtier de stator (4) et le boulon fileté (14).

3. Unité d'alignement (2) selon la revendication 1 ou 2, **caractérisée en ce qu'**une seconde fente axiale est formée entre le support (30) et le boîtier de stator (4) et l'unité d'alignement (2) présente un boîtier extérieur (40) dans lequel le boîtier de stator (4) est reçu, et le boîtier extérieur (40) est conçu de sorte qu'il recouvre la seconde fente axiale entre le support (30) et le boîtier de stator (4).

4. Unité d'alignement (2) selon l'une quelconque des revendications 1 à 3, **caractérisée en ce que** le boulon fileté (14) présente une géométrie d'attaque d'outil (22), de préférence réalisée sur son côté intérieur, en particulier sous la forme d'un six pans intérieur, pour l'introduction d'un couple de rotation par l'intermédiaire d'une première clé à douille (56) et/ou le support (30) présente une géométrie d'attaque d'outil, de préférence réalisée sur son côté extérieur, pour l'introduction d'un couple de rotation par l'intermédiaire d'une seconde clé à douille.

5. Unité d'alignement (2) selon l'une quelconque des revendications 1 à 4, **caractérisée en ce que** l'unité d'alignement (2) présente une douille filetée (10) réalisée séparément du boîtier de stator (4) et solidaire du boîtier de stator (4), qui forme une section de filetage intérieur (12) par laquelle la section de filetage extérieur (18) du boulon fileté (14) est connectée au boîtier de stator (4).

6. Unité d'alignement (2) selon la revendication 5, **caractérisée en ce que** la douille filetée (10) présente au moins un ergot (42) faisant saillie radialement qui s'engage dans le boîtier de stator (4), en particulier dans un évidement correspondant dans le boîtier de stator (4), pour une connexion bloquée en rotation par complémentarité de formes et/ou bloquée axialement par complémentarité de formes avec le boîtier de stator (4).

7. Unité d'alignement (2) selon l'une quelconque des revendications 1 à 6, **caractérisée en ce que** l'unité d'alignement (2) présente un arbre de rotor (44) pouvant être connecté à l'unité d'entraînement par transmission de couple, arbre qui pénètre axialement par sections dans le boulon fileté (14) et présente sur son côté extérieur une géométrie d'attaque d'outil (52), en particulier sous la forme d'un six pans extérieur, pour l'introduction d'un couple de rotation par l'intermédiaire d'une clé à douille, de préférence par l'intermédiaire de la première clé à douille (56).

8. Unité d'alignement (2) selon la revendication 7, **caractérisée en ce que** l'arbre de rotor (44) présente une sortie de la géométrie d'attaque d'outil (54) se rétrécissant en forme d'entonnoir.

9. Unité d'alignement (2) selon la revendication 7 ou 8, **caractérisée en ce que** l'unité d'alignement (2) présente une première clé à douille (56) pour l'introduction du couple de rotation dans la géométrie d'attaque d'outil (22) du boulon fileté (14) et pour l'introduction du couple de rotation dans la géométrie d'attaque d'outil (52) de l'arbre de rotor (44) qui présente une section d'attaque (58) en forme de corps creux, sur le côté extérieur duquel une première contre-section (60) complémentaire à la géométrie d'attaque d'outil (22) du boulon fileté (14) est formée, en particulier sous la forme d'un six pans extérieur, et sur le côté intérieur duquel une seconde contre-section (62) complémentaire à la géométrie d'attaque d'outil (52) de l'arbre de rotor (44) est formée, en particulier sous la forme d'un six pans intérieur.

10. Procédé de montage pour le montage d'une unité d'alignement (2) pour un instrument médical selon l'une quelconque des revendications 1 à 9, dans lequel
- le boulon fileté (14) est vissé dans le boîtier de stator (4) jusqu'à ce que le boulon fileté (14) repose axialement contre le boîtier de stator (4),
- le support (30) est vissé sur le boîtier de stator (4) jusqu'à ce que le support (30) repose axialement contre le boulon fileté (14),
- le support (30) est dévissé du boîtier de stator (4) jusqu'à ce que le support (30) présente un alignement radial prédéterminé par rapport au boîtier de stator (4),
- le boulon fileté (14) est dévissé du boîtier de stator (4) jusqu'à ce que le boulon fileté (14) repose axialement contre le support (30), et
- le support (30) et le boulon fileté (14) tournent dans des sens opposés l'un par rapport à l'autre.
